# EUROPEAN PATENT APPLICATION

(11) **EP 3 748 008 A1**
(43) Date of publication of application: **09.12.2020**
(21) Application number: 20178681.1
(22) Date of filing: 08.06.2020
(51) Int. Cl.: C12P 7/62, C12P 41/00, C12N 9/20

(54) **ENANTIOSELECTIVE ACETYLATION OF R/S-PANTOLACTONE**

(30) Priority: 07.06.2019 EP 19178974
(71) Applicant: DSM IP Assets B.V., 6411 TE Heerlen (NL)
(72) Inventor: DE JONG, René, Marcel, 4303 Kaiseraugst (CH); MEDLOCK, Jonathan, Alan, 4303 Kaiseraugst (CH); MUELLER, Monika, 6167 RD Geleen (NL); CALLANT, Dominique, Monique, Charles, 6167 RD Geleen (NL); VERMOTE, Linda, Andrea, Laura, 6167 RD Geleen (NL); SCHÜRMANN, Martin, 6167 RD Geleen (NL)
(74) Representative: Seibel-Thomsen, Nadja

(57) **Abstract**

The present invention relates to formation of highly enriched (R)-pantolactone acetate from conversion of (R/S)-pantolactone in a process comprising (1) kinetic resolution using variants of Candida antarctica lipase A (CalA) with variations at positions 211, 223, 326 and 327 as an enantioselective acetylating enzyme and (2) racemization of (S)-pantolactone in the presence of at least one specific chemical catalyst which is ruthenium transition metal complex "Shvo catalyst", wherein both steps might be carried-out in a one-pot reaction /system.

## Description

The present invention relates to formation of highly enriched (R)-pantolactone acetate from conversion of (R/S)-pantolactone in a process comprising (1) kinetic resolution using an enantioselective acetylating enzyme and (2) racemization of (S)-pantolactone in the presence of at least one specific chemical catalyst, wherein both steps might be carried-out in a one-pot reaction /system.

Pantothenate or vitamin B5 is a member of the B complex of vitamins which is essential for mammals including humans. It has to be supplied as food or feed additive. In cells, pantothenate is used primarily for the biosynthesis of coenzyme A and acyl carrier protein. These essential coenzymes function in the metabolism of acyl moieties, which form thioesters with the sulfhydryl group of the 4'-phosphopantethein portion of these molecules. Importantly, only (R)-pantothenate is biologically active as vitamin.

Pantothenate has been synthesized conventionally via chemical synthesis from bulk chemicals. However, the substrates required for chemical synthesis are expensive and the racemic intermediates have to be optically resolved. Bacterial or microbial systems, if available, that produce enzymes useful in pantothenate biosynthesis processes, are not yet feasible for industrial production because of insufficient yield and/or selectivity of the used enzymes.

Thus, there is a need for more effective and simpler methods towards enantioselective production of (R)-pantolactone to be used in an industrial scale, with no need for further resolution and/or purification steps.

Surprisingly, we now identified a process wherein racemic pantolactone together with an acetate ester can be converted into pantolactone acetate, with a percentage of up to 100% in the (R)-conformation, said process comprising the steps of (1) enantioselective alcoholysis of (R/S)-pantolactone and an acetate ester using (R)-selective enzymes and (2) racemization of (S)-pantolactone using a specific transition metal complex as (chemical) catalyst. The (R)-pantolactone acetate can be further converted into (R)-pantolactone by methods known in the art.

Particularly, the present invention is directed to (1) a process for conversion of racemic (R/S)-pantolactone via catalytic action of enzymes having carboxylic ester hydrolase [EC 3.1.1] activity, wherein (R/S)-pantolactone and an acetate ester are selectively converted into (R)-pantolactone-acetate via enantioselective alcoholysis.

For the purpose of the present invention, i.e. enantioselective alcoholysis of racemic pantolactone and an acetate ester into (R)-pantolactone acetate as of step (1) and as described herein, any enzyme of class [EC 3.1.1] isolated from various sources can be used, particularly such enzymes isolated from fungi including yeast, bacteria or higher eukaryotes. Preferably, the enzyme is selected from the class of lipases [EC 3.1.1.3].

The enzymes according to [EC 3.1.1.3] are naturally involved in hydrolysis of ester bonds in lipids and wax esters, but can also catalyze hydrolysis, acidolysis, alcoholysis and transesterification reactions using a wide diversity of ester bond containing substrates used in chemical synthesis.

In one embodiment, the enantioselective enzyme to be used in step (1) and as defined herein is selected from Candida, preferably Candida antarctica lipase A (CalA), more preferably a polypeptide with at least about 80%, such as e.g. 85, 90, 95, 98 or 100% identity to SEQ ID NO:1, including polynucleotides encoding said polypeptide such as e.g. the polynucleotide of SEQ ID NO:2. CalA is commercially available from various suppliers, e.g. such as Novozymes or Merck.

The term "lipase", or "enzyme having lipase activity", "pantolactone hydrolyzing enzyme", "alcoholysis enzyme", "enantioselective enzyme", "(R)-selective enzyme" or "(R/S)-pantolactone transesterase" are used interchangeably herein. It refers to an enzyme having lipase activity which is involved in conversion, i.e. enantioselective alcoholysis/acetylation, of (R/S)-pantolactone and an acetate ester into (R)-pantolactone acetate as defined herein. The terms "CalA" and "Candida antarctica lipase A" are used interchangeably herein.

Suitable acetate esters to be used for acetylation of (R/S)-pantolactone are selected from either unsaturated, saturated or aromatic acetates including but not limited to vinyl acetate, phenylvinyl acetate, ethoxyvinyl acetate, isoprenyl acetate, isopropenyl acetate, ethyl acetate or p-chlorophenyl acetate, preferably vinyl acetate.

The enzymatic conversion of (R/S)-pantolactone and an acetate ester as defined herein into (R)-pantolactone acetate, i.e. enantioselective alcoholysis, as described herein can be performed with isolated enzymes, such as e.g. carboxylic ester hydrolases or alcoholysis enzymes as defined herein, particularly lipases, such as e.g. CalA, that might be expressed in a suitable host cell, such as e.g. yeast or bacteria, preferably Pichia or Escherichia, such as e.g. as heterologous enzymes. Particularly, the enantioselective enzymes as described herein are heterologous expressed in Pichia pastoris. Isolation of suitable enzymes can be done by known methods, the respective enzymes might be further used in isolated form or in the form of a cell extract, powder or liquid formulations, i.e. in immobilized or non-immobilized form. Preferably, the enzymes - either modified or non-modified - as defined herein, such as e.g. CalA, that are used in a process for enantioselective acetylation of (R/S)-pantolactone and an acetate ester as defined herein, are used in isolated form, such as e.g. in liquid or immobilized form, such as either covalently bound or adsorbed, e.g. on epoxy carrier, depending on the supplier. When using immobilized forms, the enzymes might be used several times with more or less the same performance (so-called recycling). The described enzyme forms apply to both the modified and non-modified enzymes.

The terms "conversion", "alcoholysis", "acetylation", "transesterification", "enantioselective acetylation" or "enantioselective alcoholysis" in connection with step (1), i.e. enzymatic catalysis of the enzymes [EC 3.1.1.3] as described herein, particularly [EC 3.1.1.3], leading to enantioselective production of (R)-pantolactone acetate from racemic pantolactone and an acetate ester as defined herein, are used interchangeably herein. As used herein, the term "conversion rate" refers to the activity of the lipase, particularly CalA, which can be measured as conversion of (R/S)-pantolactone into pantolactone acetate. The term "E value" (E) as used herein refers to the general enantioselectivity of the enzyme independent of the conversion rate. The "enantiomeric excess" (ee) as used herein refers to the degree to which a sample contains one enantiomer (such as e.g. (R)-pantolactone) in higher amount than the other enantiomer (such as e.g. (S)-pantolactone), see Helpfile on selectivity, Uni Graz, 2012: http://biocatalysis.uni-graz.at/enantio/DataFiles/Selectivity-Help.pdf.

The term "racemic (R/S)-pantolactone" refers to a mixture comprising (R) and (S)-pantolactone in any ratio, including a mixture wherein the (R) and (S)-enantiomers are present in equal quantities, i.e. a so-called racemate.

As used herein, the term "specific activity" or "activity" with regards to enzymes means its catalytic activity, i.e. its ability to catalyze formation of a product from a given substrate. The specific activity defines the amount of substrate consumed and/or product produced in a given time period and per defined amount of protein at a defined temperature. Typically, specific activity is expressed in µmol substrate consumed or product formed per min per mg of protein. Typically, µmol/min is abbreviated by U (= unit). Therefore, the unit definitions for specific activity of µmol/min/(mg of protein) or U/(mg of protein) are used interchangeably throughout this document. An enzyme is active, if it performs its catalytic activity in vivo, i.e. within the host cell as defined herein or within an in vitro system in the presence of a suitable substrate. The skilled person knows how to measure enzyme activity, in particular activity of alcoholysis enzymes as defined herein, such as in particular CalA activity. Analytical methods to evaluate the capability of a suitable enzyme as defined herein for formation of (R)-pantolactone acetate from alcoholysis of (R/S)-pantolactone and an acetate ester as defined herein are known in the art.

The present invention is directed to a process according to step (1) for enantioselective alcoholysis of (R/S)-pantolactone, wherein the (R/S)-pantolactone and an acetate ester as defined herein is enzymatically converted into pantolactone acetate and (S)-pantolactone with a conversion rate in the range of at least about 20, such as e.g. at least about 25, 30, 35, 40, 45, 50, 55, 60, 65 or up to 70% conversion of racemic pantolactone into pantolactone acetate. Preferably, the pantolactone acetate is present as (R)-enantiomer, with preferred E values greater than about 1, such as e.g. 1.5, 2, 3, 5, 6, 8, 9, 10, 15, 20, 25, 30, 40, 50, 60 or even 100 and more.

In a particular embodiment, the process according to the present invention comprising step (1) and step (2) leads to a conversion of (R/S)-pantolactone and an acetate ester as defined herein into (R)-pantolactone acetate with a conversion rate of at least about 20, such as e.g. at least about 25, 30, 35, 40, 45, 50, 55, 60, 65, 70, 75, 80, 85, 90, 95 or even up to 100% and E values greater than about 1, such as at least about 1.5, 2, 3, 5, 6, 8, 10, 15, 20, 25, 30, 40, 50, 60 or even 100 and more.

In a way of further improving the enantioselectivity towards (R)-pantolactone acetate according to step (1) and as defined herein, we surprisingly found that modifications in the substrate binding region of CalA, preferably a polypeptide with at least about 80% identity to SEQ ID NO:1, could lead to increased conversion rate and/or E-values, particularly via introduction of one or more amino acid substitution(s) in the substrate binding region of the enzyme, preferably in a sequence corresponding to amino acid residues T211 to V327 in the polypeptide according to SEQ ID NO:1, more particularly via introduction of one or more amino acid substitution(s) at position(s) corresponding to residue(s) selected from the group consisting of position 211, 223, 326, 327, and combinations thereof in the polypeptide with at least about 80%, such as e.g. 85, 90, 95, 98 or 100% identity to SEQ ID NO:1.

The use of such modified CalA in a process for enantioselective conversion of (R/S)-pantolactone and an acetate ester as defined herein into (R)-pantolactone acetate, i.e. step (1), leads to conversion rates in the range of at least about 20 to 67% with E values in the range of at least about 2 to 9.

As used herein, a "modified" enzyme, i.e. modified lipase, has a preferred activity and/or specificity towards enantioselective formation of (R)-pantolactone acetate compared to the corresponding reaction catalyzed by a non-modified enzyme. Preferred enzyme is CalA. A "non-modified" enzyme as defined herein, particularly CalA, as used herein refers to the respective endogenous or commercially available enzymes not carrying one or more amino acid substitution(s) as defined herein.

As used herein, a host cell carrying/expressing a modified catalytic activity as defined herein, particularly CalA, comprising one or more amino acid substitution(s) as defined herein, is referred to as "modified" host cell. The respective host cell carrying a non-modified catalytic activity, such as encoding the wild-type CalA gene, is referred to as "non-modified" host cell.

In one embodiment, the modified enzyme as defined herein, in particular enzyme with modified CalA activity, comprises an amino acid substitution at a position corresponding to residue 211 in the polypeptide according to SEQ ID NO:1, preferably substitution of threonine by lysine or glutamine (T211K or T211Q), more preferably T211Q. Using said modified CalA enzyme in step (1) as defined herein, conversion rates in the range of at least about 30 to 40 and E value of at least about 2 could be achieved, corresponding to ee for (R)-pantolactone acetate of at least about 28, which is at least about 2x compared to a strain expressing the respective non-modified enzyme, including but not limited to CalA according to e.g. SEQ ID NO:1.

In one embodiment, the modified enzyme as defined herein, in particular modified CalA activity, comprises an amino acid substitution at a position corresponding to residue 223 in the polypeptide according to SEQ ID NO:1, preferably substitution of phenylalanine by histidine (F223H). Using said modified CalA enzyme in step (1) as defined herein, conversion rates in the range of at least about 27 and E value of at least about 9 could be achieved, corresponding to ee for (R)-pantolactone acetate of at least about 78, which is at least about 6-7x compared to a strain expressing the respective non-modified enzyme, including but not limited to CalA according to e.g. SEQ ID NO:1.

In a further embodiment, the modified enzyme as defined herein, in particular modified CalA activity, comprises an amino acid substitution at a position corresponding to residue 326 in the polypeptide according to SEQ ID NO:1, preferably substitution of isoleucine by valine, serine, glutamine or alanine (1326V, 1326S, 1326Q, 1326A), more preferably 1326V or I326S. Using said modified CalA enzyme in step (1) as defined herein, conversion rates in the range of at least about 20, 30 or 40 and E value of at least about 3 or 4 could be achieved, corresponding to ee for (R)-pantolactone acetate of at least about 27 or up to 40 or 54, which is at least about 2x compared to a strain expressing the respective non-modified enzyme, including but not limited to CalA according to e.g. SEQ ID NO:1.

In one embodiment, the modified enzyme as defined herein, in particular modified CalA, comprises an amino acid substitution at a position corresponding to residue 327 in the polypeptide according to SEQ ID NO:1, preferably substitution of valine by serine, threonine, asparagine or alanine (V327S, V327T, V327N, V327A), more preferably V327A, V327N, V327T. Using said modified CalA enzyme in step (1) as defined herein, conversion rates in the range of at least about 20, 40, 50 or 60 and E value of at least about 2 could be achieved, corresponding to ee for (R)-pantolactone acetate of at least about 21, 25 or 32, which is at least about 2x compared to a strain expressing the respective non-modified enzyme, including but not limited to CalA according to e.g. SEQ ID NO:1.

The described amino acid substitution(s) at a position corresponding to residues as disclosed herein might be combined with each other, leading to conversion rates and/or an E value which are increased as compared to single mutations, such as e.g. an increase in the range of at least about 2, 5, 10, 20, 50% compared to single mutations. Selectivity mutations in enzyme active sites often have an additive or preferably synergistic effect when they are combined, thus increasing the overall selectivity of the enzyme. Thus, combination of the selectivity mutations described herein might improve the E value of the enzymes even more, such as e.g. leading to E values of about 10 or more and/or conversion rates in the range of about 50% or more.

The generation of a mutation into nucleic acids or amino acids, i.e. mutagenesis, may be performed in different ways, such as for instance by random or site-directed mutagenesis, physical damage caused by agents such as for instance radiation, chemical treatment, or insertion of a genetic element. The skilled person knows how to introduce mutations.

In one aspect of the present invention, a host cell comprising and expressing a modified or non-modified enantioselective enzyme as defined herein, such as e.g. modified or non-modified CalA activity, is used in a process for production of (R)-pantolactone acetate from conversion of racemic R/S-pantolactone and an acetate ester as defined herein. The host cell comprising the modified/non-modified enzyme may be cultured in an aqueous medium supplemented with appropriate nutrients under aerobic or anaerobic conditions and as known by the skilled person for the respective host cells. Optionally, such cultivation is in the presence of proteins and/or co-factors involved in transfer of electrons, as known in the art. The cultivation/growth of the host cell may be conducted in batch, fed-batch, semi-continuous or continuous mode. (R)-pantolactone acetate might be isolated and/or optionally further purified from the mix of (R)-pantolactone acetate and (S)-pantolactone.

In one embodiment of the invention, step (1) as described herein is carried out using an immobilized modified or non-modified enzyme for enantioselective alcoholysis of (R/S)-pantolactone with an acetate ester to mainly form (R)-pantolactone-acetate. Such process might be performed under conditions known in the art, e.g. such as in an aqueous medium. Particularly, said process according to step (1) is performed under conditions which are not interfering with the racemization of (S)-pantolactone of step (2). Examples of suitable solvents which could be used in step (1) include but are not limited to aliphatic hydrocarbons, aromatic hydrocarbons, chlorinated hydrocarbons, esters, ethers, and/or alcohols, particularly under conditions comprising at least about 1% of water.

With regards to the present invention, it is understood that organisms, such as e.g. microorganisms, fungi, algae or plants also include synonyms or basonyms of such species having the same physiological properties, as defined by the International Code of Nomenclature of Prokaryotes or the International Code of Nomenclature for algae, fungi, and plants (Melbourne Code).

The present invention is directed to a 2-step process, wherein (R/S)-pantolactone and an acetate ester are selectively converted into (R)-pantolactone-acetate via enantioselective alcoholysis and as described above as step (1) and wherein the by-product (S)-pantolactone is converted back into (R/S)-pantolactone in the presence of one or more specific transition metal complex(es) as (chemical) catalyst(s) and as described herein as step (2).

As used herein, the term "conversion" in connection with step (2) refers to the activity of the transition metal complex used for selective racemization of (S)-pantolactone into (R/S)-pantolactone. The term "racemization" is art-recognized and refers to conversion of either (R)- or (S)-enantiomers in a racemic form of (R/S)-isomers. As used herein, it refers to the selective conversion of (S)-pantolactone into (R/S)-pantolactone.

In one embodiment, the present invention is directed to a process comprising step (1) and step (2), wherein both steps are performed in one reaction, i.e. a "one-pot reaction". They might be alternatively performed in a loop or cascade reactor. In case step (1) and (2) are performed in a loop or cascade reactor, the temperature in step (1) and (2) might vary, e.g. with step (1) being carried out at about 40°C and step (2) being carried out at higher temperature, e.g. at least about 50°C.

In one embodiment, the present invention is directed to a process comprising step (1) and step (2), wherein both steps are combined in a loop or cascade reactor containing an area where step (1) occurs and a subsequent area where step (2) occurs.

Particularly, the racemization of (S)-pantolactone according to step (2) is performed under conditions which are not interfering with the enzymatic acetylation of step (1) and vice-versa.

In a particular embodiment, the racemization of step (2) and as described herein is carried out in the presence of at least one specific transition metal complex as (chemical) catalyst, wherein the transition metal(s) is/are selected from the group consisting of Ru, Rh, Ir, Fe, Mn and Co, preferably selected from Ru or Ir. The at least one transition metal complex might comprise one or more ligands.

In a preferred embodiment, a catalyst to be used in step (2) and as defined herein is the "Shvo catalyst", which is 1-hydroxytetraphenyl-cyclopentadienyl(tetraphenyl-2,4-cyclopentadien-1-one)-µ-hydrotetracarbonyldiruthenium(II). This catalyst was first described by Shvo et al. (J.Am.Chem.Soc., 1986, 108, pp. 7400-7402).

A suitable solvent for the racemization as of step (2) and as described herein might be selected from aliphatic hydrocarbons, aromatic hydrocarbons, chlorinated hydrocarbons, esters, ethers, and/or alcohols, preferably selected from THF, 2-methyl-THF, toluene, xylene, cyclohexane, and/or ethyl acetate, either in the absence or in the presence of at least about 1% of water. Solvents to be used in a one-pot reaction as described herein wherein step (1) is performed in the presence of immobilized enzymes might be selected from organic solvents as described above, such as e.g. toluene, dichloromethane, acetone, methyl-tert-butylether (MTBE), ethyl acetate and the like, particularly under conditions comprising at least about 1% of water.

The process according to step (2) and as described herein might be carried out at elevated temperatures, such as e.g. temperatures up to 100°C, such as e.g. between about 25 to 100°C, preferably between about 40 to 90°C. With regards to step (1) and (2) carried out preferably in a one-pot reaction, the temperature might be selected from about 25 to 80°C, preferably from about 30 to 70°C, but particularly at least about 50°C or higher.

The process comprising step (1) and (2) as described herein is used for production of (R)-pantolactone acetate. The (R)-pantolactone acetate is furthermore hydrolyzed to (R)-pantolactone and converted to pantothenic acid and its derivatives by well-known transformations.

The following examples are illustrative only and are not intended to limit the scope of the invention in any way.

### Examples

### Example 1: General methods, media, strains and plasmids

All basic molecular biology and DNA manipulation procedures described herein were generally performed according to Sambrook et al. (1989. Molecular Cloning: A Laboratory Manual. Cold Spring Harbor Laboratory Press: New York) or Ausubel et al. (1998. Current Protocols in Molecular Biology. Wiley: New York). For the media listed in Table 1, the following components were used:
1M KPi pH 6.0: autoclave;
134 g/l yeast nitrogen base (YNB) without amino acids (10x): filter sterilize and store at 4°C;
0.2 g/l Biotin (500x): filter sterilize and store at 4°C;
40% glucose: autoclave;
10% glycerol: autoclave.

**Table 1: Media.**

| Medium | Components |
|---|---|
| YPD | 10 g/l yeast extract (final) |
| | 20 g/l bacto peptone (final) |
| | Autoclave |
| | add 50 ml/l 40% glucose (final concentration 2%) |
| BMGY | 10 g/l yeast extract (final) |
| | 20 g/l bacto peptone (final) |
| | Autoclave |
| | add 100 ml/l 10% glycerol (final 1%) |
| | add 100 ml/l 10% glycerol (final 1%) |
| | add 100 ml/l 1M KPi pH 6.0 (final 100 mM) |
| | add 2 ml/l 500x Biotin |
| BMMY | 10 g/l yeast extract (final) |
| | 20 g/l bacto peptone (final) |
| | Autoclave |
| | add 10 ml/l methanol (final 1%) |
| | add 100 ml/l 10x YNB (final 1x) |
| | add 100 ml/l 1M KPi pH 6.0 (final 100 mM) |
| | add 2 ml/l 500x Biotin |

### Example 2: Expression of CalA mutants in Pichia pastoris

Gene encoding CalA variants, i.e. modified CalA, were ordered as synthetic DNA at ATUM (Newark, California, US). The genes encoding the CalA variants were cloned to the methanol inducible Pichia pastoris expression vector pD902, whereby the wildtype calA gene is fused to the α-mating factor of Saccharomyces cerevisiae, according to the manufacturer's instructions with slight variations as follows:
Single colonies from a selection plate were inoculated into 2.5 ml BMGY (200 rpm, 25°C). On the 5^{th} day, 2.083 ml BMMY was added to each well, with further shaking at 200 rpm (30°C). After 6 h, 21 µl MeOH was added to each well. This addition was repeated again after further 16, 24, 40, and 48 h. After 8 days in total, the cells were harvested (10 min, 1000xg, 4°C), the supernatant transferred to an Eppendorf tube and stored at 4°C.

For detection of active protein in the culture supernatant, activity of the variants on the model substrate para-nitrophenyl decanoate (p-NPD) was measured. The assay monitors the product formation of para-nitrophenol (p-NP) over time using a UV/VIS spectrophotometer.

For determination of the molar extinction coefficient (ε) of the formed product (p-NP), a calibration curve has to be measured for this compound. A stock solution for p-NP of 10 mmol/l is required to make the standards. Standards of various p-NP concentration (0, 50, 500, 1000, 1429, 2000 µM) in water are prepared. The standards are measured in a 1ml cuvette containing 1ml (3-(N-morpholino)propanesulfonic acid (MOPS) buffer (0.1 M pH = 7.5) pre-heated to 37°C and 40µl standard solution. Measurements are done at 37°C, 405 nm using a Perkin Elmer Lambda 35 UV/VIS spectrophotometer. For measurement of enzymatic samples, 40 µl of enzyme was mixed with 900 µl MOPS (0.1 M pH = 7.5; pre-heated to 37°C) and reaction was initiated by addition of 100 µl p-NPD stock solution (2.5 mM in Dimethylsulfoxide (DMSO)). Increase of absorbance is measured for 5 minutes at 405 nm. Calculation of enzyme activity is done as follows: definition for a unit (U) is the amount of enzyme that releases 1 µmol of p-NP per minute under these reaction conditions (37°C, pH 7.5).

### Example 3: Enantioselective acetylation of (R/S)-pantolactone according to step (1)

Formation of R-pantolactone acetate was tested with the best CalA-variants (see Table 2). Reactions were performed in HPLC-vials with a final volume of 1125 µl. The enzyme supernatants (1500 µl or 750 µl, respectively) were freeze dried, 375 µl of 200 mM (RS)-PL in vinyl acetate was added and the reaction was further diluted with 750 µl vinyl acetate to a final concentration of 67 mM pantolactone. S/E ratios in the reactions were 52 (750 µl enzyme) or 26 (1500 µl enzyme). Incubations were done at 28°C, shaken. 50 µl samples were taken and diluted with 450 µl of acetonitrile with internal standard. The results are shown in Table 2, showing the conversion rate in % ("activity"), the E value ("selectivity") and the ee in %. The position of the mutations corresponds to the position in SEQ ID NO:1. Enzyme-concentration in each sample was standardized, i.e. each reaction was run with about the same concentration of CalA.

**Table 2:Improved (R)-selectivity of CalA variants. "ee R-PLA" means ee of R-pantolactone acetate (for E calculation).**

| Code | mutation | activity | selectivity | ee R-PLA |
|---|---|---|---|---|
| M02 | T211K | 31.1 | 2 | -23.4 |
| M03 | T211Q | 41.4 | 2 | 28.0 |
| M05 | F223H | 26.8 | 9 | 77.7 |
| M07 | 1326V | 39.4 | 4 | 53.5 |
| M08 | 1326S | 32.9 | 3 | 41.0 |
| M10 | 1326Q | 21.2 | 2 | 27.7 |
| M12 | V327S | 43.7 | 2 | 21.3 |
| M14 | V327T | 66.9 | 2 | 24.9 |
| M15 | V327N | 20.9 | 2 | 25 |
| M16 | V327A | 54.3 | 2 | 32.0 |
| M21 | CalA-wt | 60.9 | 1 | 12.4 |

Measurements were done on an Agilent HP 6890 gas chromatograph equipped with hot-split injector, autosampler and FID detector. Column used was Supelco beta-Dex 225, column dimensions 30 m x 0.25 mm id x 0.5 µm df. The isothermal temperature was 140°C at an 18 minutes runtime. Helium gas was used with a ramp flow of initially 1.6 ml/min (minutes 0-7) and subsequent increase to 1.8 ml/min till the end of analysis. Injection volume was 1 µl, with a split ratio of 1 : 50 at a hot split injection at 175°C. A flame ionisation detector was used at 250°C, with a hydrogen flow of 40 mL/min, an air flow of 450 mL/min and a make-up flow of 45 mL/min.

Calibration solutions of pantolactone and pantolactone acetate, respectively, were made in concentration range of 0.5 - 5 mg/mL. A known concentration of the internal standard caprylonitrile was added, and the calibration samples were diluted with acetonitrile before injection to GC.

Enzymatic samples were diluted 4x in internal standard solution (15 mg caprylonitrile in 250 mL acetonitrile), subsequently centrifuged to precipitate protein and injected into the GC.

**Table 3:Sequence of CalA-wt.**

| SEQ ID NO: | Name of oligo or enzyme | sequence 5' to 3' |
|---|---|---|
| 1 | CalA | AALPNPYDDPFYTTPSNIGTFAKGQVIQSRKVPTDIGNANNAASFQLQYRTTN TQNEAVADVATVWIPAKPASPPKIFSYQVYEDATALDCAPSYSYLTGLDQPNK VTAVLDTPIIIGWALQQGYYVVSSDHEGFKAAFIAGYEEGMAILDGIRALKNY QNLPSDSKVALEGYSGGAHATVWATSLADSYAPELNIVGASHGGTPVSAKDTF TFLNGGPFAGFALAGVSGLSLAHPDMESFIEARLNAKGQQTLKQIRGRGFCLP QVVLTYPFLNVFSLVNDTNLLNEAPIAGILKQETVVQAEASYTVSVPKFPRFI |
| | | WHAIPDEIVPYQPAATYVKEQCAKGANINFSPYPIAEHLTAEIFGLVPSLWFI KQAFDGTTPKVICGTPIPAIAGITTPSADQVLGSDLANQLRSLNGKQSAFGKP FGPITPP |
| 2 | calA | GCTGCTTTGCCAAACCCATACGACGACCCATTTTACACTACTCCATCCAACAT CGGTACTTTCGCTAAGGGTCAGGTTATCCAGTCCAGAAAGGTTCCAACTGACA TCGGTAACGCTAACAACGCTGCTTCATTCCAGTTGCAGTACAGAACTACAAAC ACTCAGAACGAGGCTGTTGCTGACGTTGCTACTGTTTGGATTCCAGCTAAGCC AGCTTCCCCACCAAAGATCTTCTCATACCAAGTTTACGAGGACGCTACAGCTT TGGACTGTGCTCCATCTTACTCCTACTTGACTGGTTTGGACCAGCCAAACAAG GTTACTGCTGTTTTGGACACTCCAATCATCATCGGTTGGGCTTTGCAACAGGG TTACTACGTTGTTTCTTCTGACCACGAGGGTTTCAAGGCTGCTTTCATTGCTG GTTACGAAGAGGGTATGGCTATCTTGGACGGTATCAGAGCTTTGAAGAACTAC CAGAACTTGCCATCCGACTCCAAGGTTGCTTTGGAAGGTTATTCTGGTGGTGC TCATGCTACAGTTTGGGCTACTTCTTTGGCTGACTCTTACGCTCCAGAGTTGA ACATCGTTGGTGCTTCTCATGGTGGTACTCCAGTTTCTGCTAAGGACACTTTC ACTTTCTTGAACGGTGGTCCATTCGCTGGTTTTGCTTTGGCTGGTGTTTCTGG TTTGTCCTTGGCTCACCCAGACATGGAATCTTTCATCGAGGCTAGATTGAACG CTAAGGGACAGCAGACTTTGAAGCAGATCAGAGGTAGAGGTTTCTGTTTGCCA CAGGTTGTTTTGACTTACCCATTTTTGAACGTTTTCTCATTGGTTAACGACAC TAACTTGTTGAACGAAGCTCCAATCGCTGGTATCTTGAAGCAAGAGACTGTTG TTCAAGCTGAGGCTTCCTACACTGTTTCCGTTCCAAAGTTCCCAAGATTCATC TGGCACGCTATTCCAGACGAGATCGTTCCATATCAACCAGCTGCTACTTACGT TAAGGAACAGTGTGCTAAGGGTGCTAACATCAACTTCTCCCCATACCCAATTG CTGAGCACTTGACTGCTGAGATCTTCGGTTTGGTTCCATCCTTGTGGTTCATC AAGCAAGCTTTCGACGGTACTACTCCAAAGGTTATCTGTGGAACTCCTATCCC AGCTATCGCTGGAATTACTACTCCTTCCGCTGACCAAGTTTTGGGTTCCGACT TGGCTAACCAGTTGAGATCTTTGAACGGTAAGCAGTCCGCTTTCGGTAAGCCA TTCGGTCCAATTACTCCACCA |

### Example 4: Racemization of (S)-pantolactone according to step (2)

(S)-Pantolactone can be obtained from the mixture obtained in step (1) (see Ex. 3) by separation from (R)-pantolactone acetate. Alternatively, the mixture of (S)-pantolactone and (R)-pantolactone acetate from step (1) (see Ex. 3) can be used directly.

(S)-Pantolactone (310-345 mg, 2.4-2.6 mmol) was placed in a 10 ml round bottomed flask with the "Shvo catalyst" (5-10 mol%). The solvent (3 ml) was added and the mixture was inertised. The reaction mixture was heated at the described temperature for 16-20h, cooled and analysed by GC (column: Chirasil-DEX CB (25 m x 0.25 mm; Film 0.25 µm)), temperature program: 130°C (constant)). The result is shown in Table 4.

**Table 4:Reactions starting from (S) pantolactone (i.e. a mixture comprising a ratio of (R) : (S) = 2 : 98). "Exp." means "experiment", with Exp.1a given as comparative example.**

| Exp. # | Catalyst [mol%] | Temp [°C] | Solvent | Ratio (R):(S) pantolactone |
|---|---|---|---|---|
| 1a | none | 65 | THF | 2 : 98 |
| 1b | 5 | 75 | toluene | 47 : 53 |
| 1c | 5 | 75 | cyclohexane | 49 : 59 |
| 1d | 5 | 80 | toluene | 49 : 51 |
| 1e | 5 | 55 | EtOAc | 37 : 68 |
| 1f | 5 | 65 | THF | 25 : 75 |

Examples 3 and 4 can be combined together and carried out in a one-pot reaction, in a loop or cascade reactor with step (1) and (2) taking place at different sections (areas) of the loop.

## Claims

1. A process for production of (R)-pantolactone acetate comprising the steps of (1) enantioselective alcoholysis of (R/S)-pantolactone and an acetate ester using (R)-selective enzymes having lipase [EC 3.1.1.3] activity, preferably with a conversion rate of at least about 20%, and (2) racemization of (S)-pantolactone in the presence of at least one specific transition metal complex as (chemical) catalyst.

2. The process according to claim 1, wherein the enzyme of step (1) is selected from Candida antarctica, preferably CalA, more preferably an enzyme with at least about 80%, such as e.g. 85, 90, 95, 98 or 100% identity to SEQ ID NO:1.

3. An enantioselective alcoholysis enzyme catalyzing the conversion of racemic pantolactone and an acetate ester into (R)-pantolactone acetate according to step (1) in claim 1 or 2, said enzyme having lipase [EC 3.1.1.3] activity and an E value of at least about 1.5.

4. The enzyme according to claim 3, said enzyme comprising one or more amino acid substitution(s) in the substrate binding region.

5. The enzyme according to claim 4, said one or more amino acid substitution(s) are located at position(s) corresponding to residue(s) selected from the group consisting of position 211, 223, 326, 327, and combinations thereof in the polypeptide according to SEQ ID NO:1.

6. The enzyme according to claim 5, said one or more amino acid substitution(s) are located at position(s) corresponding to residue(s) selected from the group consisting of position 223, 326 and combinations thereof in the polypeptide according to SEQ ID NO:1.

7. The enzyme according to claim 6, wherein the one or more amino acid substitution(s) are selected from T211K, T211Q, F223H, I326V, I326S, I326Q, V327S, V327T, V327N, V327A, and combinations thereof, preferably F223H, I326V or 1326S, corresponding to residue(s) selected from the group consisting of position 211, 223, 326, 327, and combinations thereof in the polypeptide according to SEQ ID NO:1.

8. A host cell, preferably a yeast, more preferably Pichia, comprising a heterologous enzyme according to any one of claims 3 to 7.

9. Use of an enzyme according to any one of claims 3 to 7 or a host cell according to claim 8 in a process for production of (R)-pantolactone acetate from racemic pantolactone and an acetate ester according to step (1) in claim 1 or 2, with an E value of at least about 1.5, preferably 2-4, more preferably at least 9.

10. A process according to any one of claims 1 or 2, comprising the step of racemization of (S)-pantolactone in the presence of at least one specific transition metal complex as catalyst, preferably wherein the at least one transition metal is selected from the group consisting of Ru, Rh, Ir, Fe, Mn and Co, more preferably selected from Ru or Ir.

11. The process according to claim 10, wherein the process is carried out in a solvent selected from the group consisting of aliphatic hydrocarbons, aromatic hydrocarbons, chlorinated hydrocarbons, esters, ethers, and/or alcohols, preferably selected from the group consisting of methyl tert-butylether, THF, 2-methyl-THF, toluene, xylene, cyclohexane, and/or ethyl acetate.

12. The process according to claim 10 or 11, wherein the process is carried out at temperatures between about 25 to 80°C, preferably between about 30 to 70°C.

13. The process according to any one of claims 10 to 12, comprising racemization of (S)-pantolactone in the presence of the Shvo catalyst, which is 1-hydroxytetraphenyl-cyclopentadienyl(tetraphenyl-2,4-cyclopentadien-1 -one)-µ-hydrotetracarbonyldiruthenium(II).

14. The process according to any one of claims 1, 2 or 10 to 13, wherein both step (1) and step (2) are performed in a one-pot reactor or in a loop or cascade reactor.


SEQUENCE LISTING

<110>  DSM IP Assets B.V.

<120>  Enantioselective acetylation of R/S-pantolactone

<130>  33351-CH-NP

<160>  2

<170>  PatentIn version 3.5

<210>  1
<211>  431
<212>  PRT
<213>  Candida antarctica

<400>  1

Ala Ala Leu Pro Asn Pro Tyr Asp Asp Pro Phe Tyr Thr Thr Pro Ser
1               5                   10                  15

Asn Ile Gly Thr Phe Ala Lys Gly Gln Val Ile Gln Ser Arg Lys Val
            20                  25                  30

Pro Thr Asp Ile Gly Asn Ala Asn Asn Ala Ala Ser Phe Gln Leu Gln
        35                  40                  45

Tyr Arg Thr Thr Asn Thr Gln Asn Glu Ala Val Ala Asp Val Ala Thr
    50                  55                  60

Val Trp Ile Pro Ala Lys Pro Ala Ser Pro Pro Lys Ile Phe Ser Tyr
65                  70                  75                  80

Gln Val Tyr Glu Asp Ala Thr Ala Leu Asp Cys Ala Pro Ser Tyr Ser
                85                  90                  95

Tyr Leu Thr Gly Leu Asp Gln Pro Asn Lys Val Thr Ala Val Leu Asp
            100                 105                 110

Thr Pro Ile Ile Ile Gly Trp Ala Leu Gln Gln Gly Tyr Tyr Val Val
        115                 120                 125

Ser Ser Asp His Glu Gly Phe Lys Ala Ala Phe Ile Ala Gly Tyr Glu
    130                 135                 140

Glu Gly Met Ala Ile Leu Asp Gly Ile Arg Ala Leu Lys Asn Tyr Gln
145                 150                 155                 160

Asn Leu Pro Ser Asp Ser Lys Val Ala Leu Glu Gly Tyr Ser Gly Gly
                165                 170                 175

Ala His Ala Thr Val Trp Ala Thr Ser Leu Ala Asp Ser Tyr Ala Pro
        180                 185                 190

Glu Leu Asn Ile Val Gly Ala Ser His Gly Gly Thr Pro Val Ser Ala
    195                 200                 205

Lys Asp Thr Phe Thr Phe Leu Asn Gly Gly Pro Phe Ala Gly Phe Ala
    210                 215                 220

Leu Ala Gly Val Ser Gly Leu Ser Leu Ala His Pro Asp Met Glu Ser
225                 230                 235                 240

Phe Ile Glu Ala Arg Leu Asn Ala Lys Gly Gln Gln Thr Leu Lys Gln
                245                 250                 255

Ile Arg Gly Arg Gly Phe Cys Leu Pro Gln Val Val Leu Thr Tyr Pro
            260                 265                 270

Phe Leu Asn Val Phe Ser Leu Val Asn Asp Thr Asn Leu Leu Asn Glu
        275                 280                 285

Ala Pro Ile Ala Gly Ile Leu Lys Gln Glu Thr Val Val Gln Ala Glu
    290                 295                 300

Ala Ser Tyr Thr Val Ser Val Pro Lys Phe Pro Arg Phe Ile Trp His
305                 310                 315                 320

Ala Ile Pro Asp Glu Ile Val Pro Tyr Gln Pro Ala Ala Thr Tyr Val
                325                 330                 335

Lys Glu Gln Cys Ala Lys Gly Ala Asn Ile Asn Phe Ser Pro Tyr Pro
            340                 345                 350

Ile Ala Glu His Leu Thr Ala Glu Ile Phe Gly Leu Val Pro Ser Leu
        355                 360                 365

Trp Phe Ile Lys Gln Ala Phe Asp Gly Thr Thr Pro Lys Val Ile Cys
    370                 375                 380

Gly Thr Pro Ile Pro Ala Ile Ala Gly Ile Thr Thr Pro Ser Ala Asp
385                 390                 395                 400

Gln Val Leu Gly Ser Asp Leu Ala Asn Gln Leu Arg Ser Leu Asn Gly
                405                 410                 415

Lys Gln Ser Ala Phe Gly Lys Pro Phe Gly Pro Ile Thr Pro Pro
            420                 425                 430

<210>  2
<211>  1293
<212>  DNA
<213>  Candida antarctica

<400>  2
gctgctttgc caaacccata cgacgaccca ttttacacta ctccatccaa catcggtact      60

ttcgctaagg gtcaggttat ccagtccaga aaggttccaa ctgacatcgg taacgctaac     120

aacgctgctt cattccagtt gcagtacaga actacaaaca ctcagaacga ggctgttgct     180

gacgttgcta ctgtttggat tccagctaag ccagcttccc caccaaagat cttctcatac     240

caagtttacg aggacgctac agctttggac tgtgctccat cttactccta cttgactggt     300

ttggaccagc caaacaaggt tactgctgtt ttggacactc caatcatcat cggttgggct     360

ttgcaacagg gttactacgt tgtttcttct gaccacgagg gtttcaaggc tgctttcatt     420

gctggttacg aagagggtat ggctatcttg gacggtatca gagctttgaa gaactaccag     480

aacttgccat ccgactccaa ggttgctttg gaaggttatt ctggtggtgc tcatgctaca     540

gtttgggcta cttctttggc tgactcttac gctccagagt tgaacatcgt tggtgcttct     600

catggtggta ctccagtttc tgctaaggac actttcactt tcttgaacgg tggtccattc     660

gctggttttg ctttggctgg tgtttctggt ttgtccttgg ctcacccaga catggaatct     720

ttcatcgagg ctagattgaa cgctaaggga cagcagactt tgaagcagat cagaggtaga     780

ggtttctgtt tgccacaggt tgttttgact tacccatttt tgaacgtttt ctcattggtt     840

aacgacacta acttgttgaa cgaagctcca atcgctggta tcttgaagca agagactgtt     900

gttcaagctg aggcttccta cactgtttcc gttccaaagt tcccaagatt catctggcac     960

gctattccag acgagatcgt tccatatcaa ccagctgcta cttacgttaa ggaacagtgt    1020

gctaagggtg ctaacatcaa cttctcccca tacccaattg ctgagcactt gactgctgag    1080

atcttcggtt tggttccatc cttgtggttc atcaagcaag ctttcgacgg tactactcca    1140

aaggttatct gtggaactcc tatcccagct atcgctggaa ttactactcc ttccgctgac    1200

caagttttgg gttccgactt ggctaaccag ttgagatctt tgaacggtaa gcagtccgct    1260

ttcggtaagc cattcggtcc aattactcca cca                                 1293